# EUROPEAN PATENT SPECIFICATION

(11) **EP 3 117 802 B1**
(45) Date of publication and mention of the grant of the patent: **03.01.2024**
(21) Application number: 15761481.9
(22) Date of filing: 27.02.2015
(51) Int. Cl.: A61F 2/82, A61F 2/24, A61F 2/95

(54) **STENT WITH MECHANICAL OR BIOLOGICAL HEART VALVE FOR MINIMALLY INVASIVE VALVE REPLACEMENT PROCEDURE, AND APPLICATOR**
STENT MIT MECHANISCHER ODER BIOLOGISCHER HERZKLAPPE FÜR MINIMAL INVASIVES KLAPPENERSATZVERFAHREN UND APPLIKATOR
STENT AVEC VALVULE CARDIAQUE MÉCANIQUE OU BIOLOGIQUE POUR PROCÉDÉ D'ÉCHANGE VALVULAIRE MINI-INVASIF, ET DISPOSITIF DE MISE EN PLACE

(30) Priority: 14.03.2014 BR 1406114
(43) Date of publication of application: 18.01.2017
(73) Proprietor: Neves Filho, Antonio Francisco, 12421-550 Pindamonhangaba - SP (BR)
(72) Inventor: Neves Filho, Antonio Francisco, 12421-550 Pindamonhangaba - SP (BR)
(74) Representative: Feltrinelli, Secondo Andrea
(86) International application number: PCT/BR2015/050024
(87) International publication number: WO 2015/135050

(56) References cited:
- EP-A1- 2 484 309
- EP-A1- 2 601 897
- WO-A1-2009/045338
- WO-A1-2012/063228
- WO-A1-2014/018173
- WO-A2-2012/023979
- WO-A2-2013/175468
- US-A1- 2005 182 486
- US-A1- 2010 312 333

## Description

The invention is a stent with mechanical or biological heart valve as defined by claim 1 and a system as defined by claim 7 including the stent of claim 1 and an application device.

This invention discloses a cardiac stent with a mechanism for positioning the mechanical or biological valve to be used during heart valve replacement or procedure, as well as a stent application device thereof. The stent has a tubular shape made of grated material with two flaps, with the heart valve fixed within. The application device comprises a syringe inside which the stent is placed, said syringe comprising a threaded cap that allows storage of the stent in a solution for conservation. Two screws pierce through the plunger of the syringe and fix the stent structure.

### DESCRIPTION OF THE STATE OF THE ART

The state of the art features a variety of devices for heart valve replacement, especially, by stents. Some of these stents feature an application device, specifically designed for the positioning procedure.

The surgical approach of mitral valve pathologies demands the application of annuloplasty rings for fixing the base, which is a relatively time-consuming procedure, since anchoring of fixing wires is needed.

Document WO2009134701 (A2) features a positioning, opening and fixing system of a device for mitral valve replacement, which comprises a grated material that, when released from the application device, opens one of the ends which adapts to the shape of the mitral valve. The end that remains in the ventricle is fixed by tension wires anchored to the myocardium. Similar constructions are featured in the US 2013/0172978 and WO 2013096541.

In this application, as well as the other mentioned documents, a specific application device was developed for this stent.

Differently, this invention comprises a cardiac stent for heart valve without using tension wires for fixing, in other words, this stent is fixed by flaps mounted in its own structure.

The state of the art features a variety of distinct approaches to the treatment of dysfunctions associated to heart valves. WO2012/063228 (A1) discloses a prosthesis for cardiovascular valve. One traditional method comprises the use of mechanical side-flow valves, such as the Starr-Edwards (initially disclosed in patent US3099016). This device consists in a closure element - usually spherical - lodged in the path of the blood flow, diverting it. The initial project envisaged numerous disadvantages, such as structural fragility of the cage stems, low resistance of the sphere (causing injury to the septa) and the need of a permanent anticoagulant medicinal therapy for the patient. The position of the valves should also be highlighted as another impacting factor on patient health.

The evolution of replacement devices took place through development of forward flow mechanical valves, comprising pivotal structures that enabled a unidirectional blood flow. However, negative aspects remained, such as mechanical fatigue, use of anticoagulant medication and calcification of the heart valve. Partial solutions for the problem of the state of the art were described in documents PI9700076-0 and US6113631.

An alternative proposal comprises the use of bioprosthesis or bioprosthetic valves, mainly built from biological tissue (bovine or pig pericardium), mimicking the leaflets that constitute human heart valves. Documents PI9202905-1 and US6358277 illustrate this concept.

On the other hand, the device revealed in document PI0711664-0 was developed to replace damaged tendinous cords in case of a mitral valve prolapse. There is no mention or any information in the report that supports its application in valve replacement. In this document, a rough connection using wires and a hook is featured. Similarly, the international application WO2012106602 features an apparatus and a positioning method, incorporating some common elements to PI0711664-0.

The application US2013079873, in turn, describes a set comprising a prosthetic valve with a valve position and support, comprising fixing means for support to the mitral annulus, providing a closing mechanism in order to avoid slipping of the chains and mentioning the possibility of the fixing device being a screw. One of the limitations inherent to this device is the impossibility of timely adjustment of chain dimensions. In case adjustments are needed, the device must be taken off, adjusted and repositioned over the valve. Further, the exposure of fixing support elements facilitates contamination or even injury of cardiac muscles.

In view of the state of the art, this invention enables a new stent and application device concept, for heart valve replacement or exchange procedures, with higher reliability and lesser duration of the procedure and equipment costs.

### DESCRIPTION OF THE DRAWINGS

Figure 1A shows that the application device may be introduced in the cardiac muscle in two ways. In both ways, the stent (3) is introduced by its application device (1 or 2), which may be positioned from the upper part of the heart, right atrium, left atrium or through the pulmonary artery (1), or, alternatively, from a cut on the lower part of the myocardium (2).
Figures 2A and 2B feature assemblies of the stents within the application devices, respectively, according to the introduction procedure of the application device (1) from the upper part (1) or the application device (2) from the lower part (2) of the myocardium. Both assemblies are needed so that the stent (3) is positioned correctly within the heart valve.
Figure 2C highlights a screw (7) that pierces through the plunger (5) of the application device (1) to connect to the stent structure (3), preferably, on the upper flap (8) of the stent.
Figure 3 shows an exploded view of the assembly of the stent (3) in which an adapter (12) is fixed, where a mechanical valve (11) is screwed or, alternatively, a biological valve (10).
Figures 4A, 4B and 4C show, respectively, the open stent (3), without a heart valve, with a biological valve (10) or a mechanical valve (11). Figure 4D shows a cross-sectional view of the stent (3) with a mechanical valve (11).

### DETAILED DESCRIPTION OF THE DRAWINGS

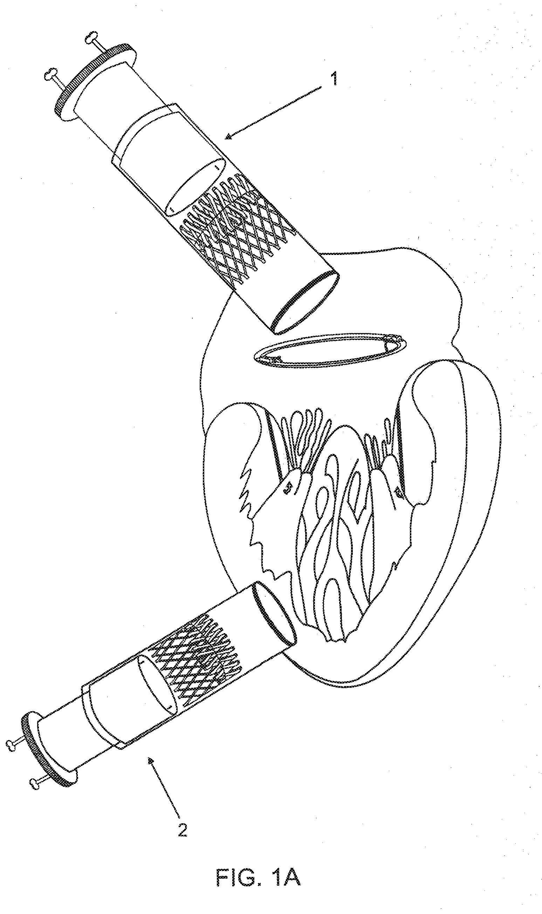

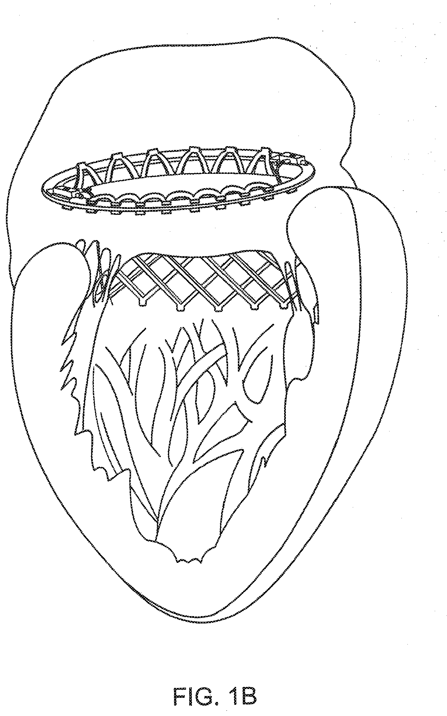

Figure 1A shows, in its upper part, the application device (1) with the stent (3) mounted within in a stretched manner. When the application device is positioned and the stent (3) is released in the myocardium valve of the patient, the stent expands so that the upper flap (8) of the stent (3) remains above the myocardium valve, while the lower flap (9) remains below the myocardium valve.

On the same Figure 1A, a second procedure option is shown with the application device (2) from the lower part of the myocardium, so that the stent (3) is introduced by the application device.

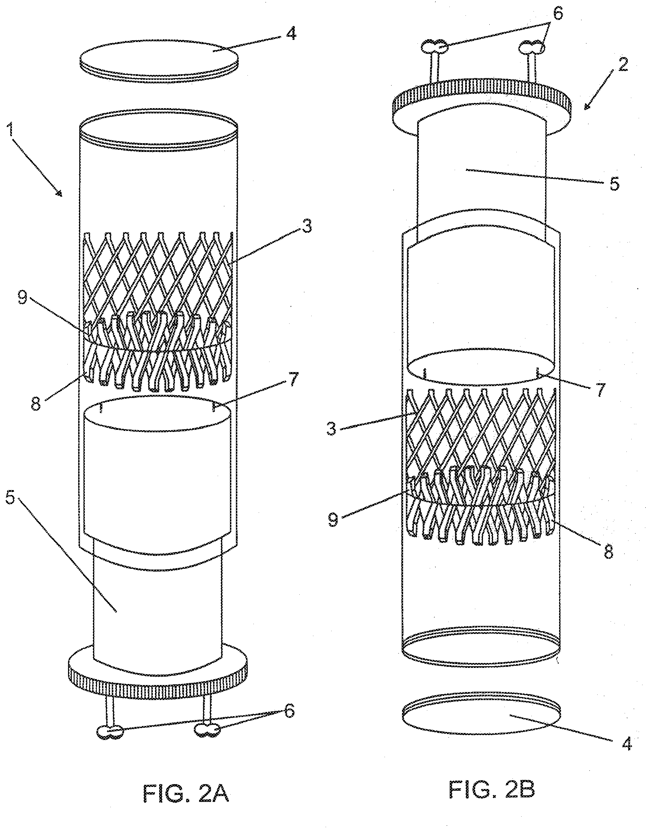

Figures 2A and 2B show the assembly of the stent (3) within application devices (1) and (2), so that the stent (3) is stretched and compressed within the application device. In assembly 2A, the stent structure (3) is through the upper flap (8) which is attached to the plunger (5) by a screw (7). In this embodiment, the application device must be introduced from the upper part of the myocardium. Figure 2B shows a configuration in which the stent (3) is positioned within the application device (2) in reverse form to the assembly of Figure 2A, which allows the same positioning of the stent (3) in the myocardium valve.

In summary, the position in which the stent (3) is assembled within the application device (1 or 2) is determined by the introduction point of the application device in the myocardium.

Additionally, the application devices (1) or (2) may be used as storage means of the stent (3). Accordingly, there is the option of a cap (4), which seals the application device, allowing it to be filled with a preservative liquid.

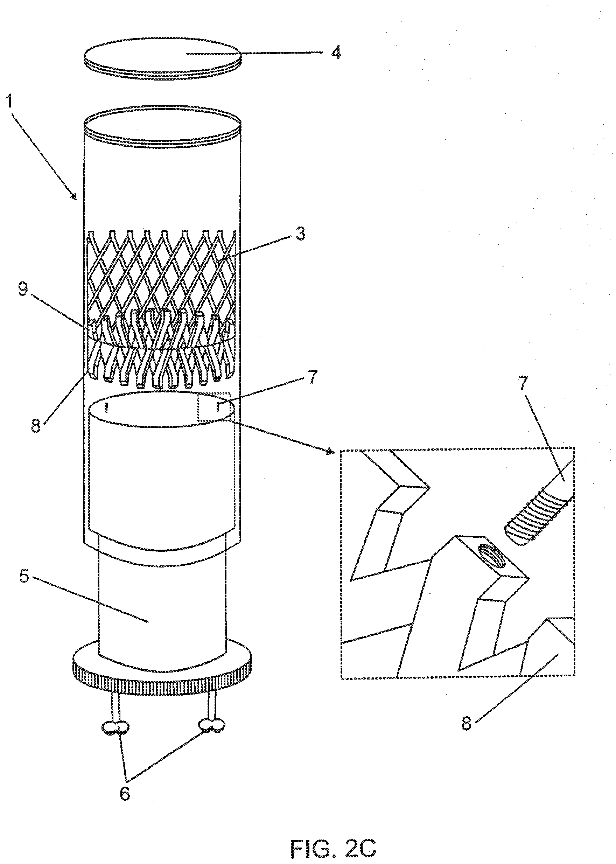

Figure 2C highlights the fixing feature of the stent (3) on the screws (7) of the application device (1 or 2) .

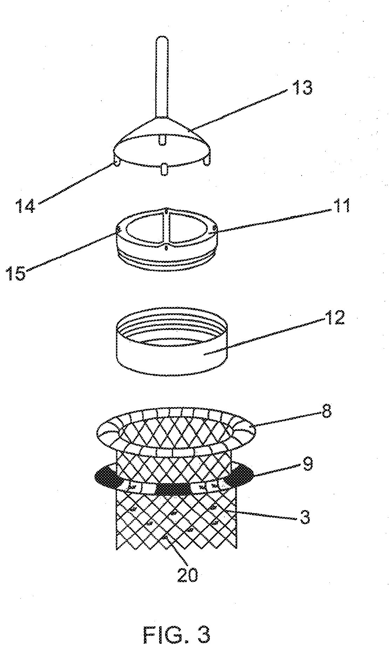

Figure 3 features an exploded view of the stent (3) in an open position, an adapter (12) with a thread and a mechanical valve (11), which is fixed and guided by the movement device (13) which is attached to the valve (11) through fixing elements (14). Optionally, the mechanical valve (11) may be replaced by a biological valve (10).

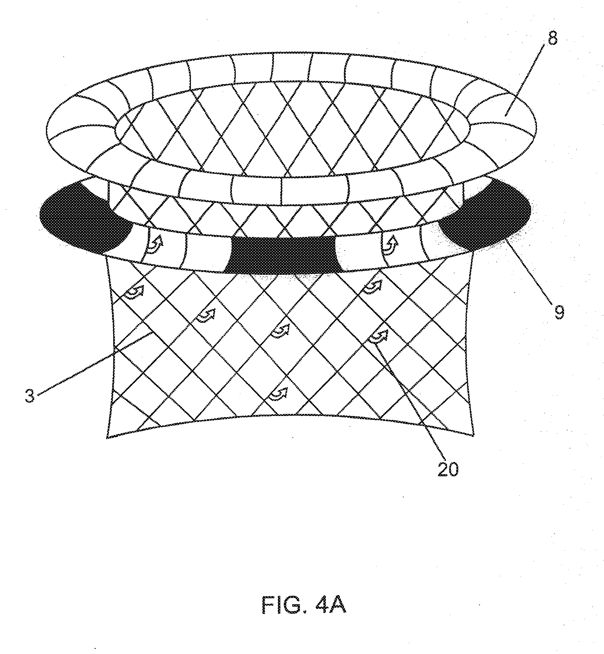

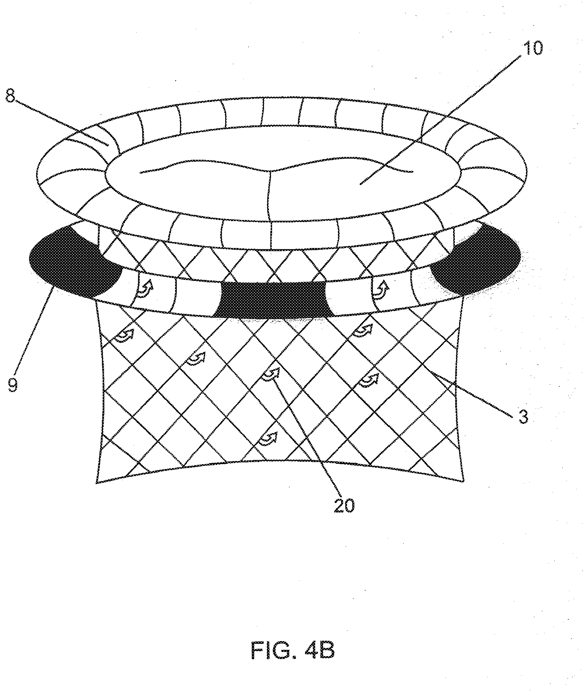

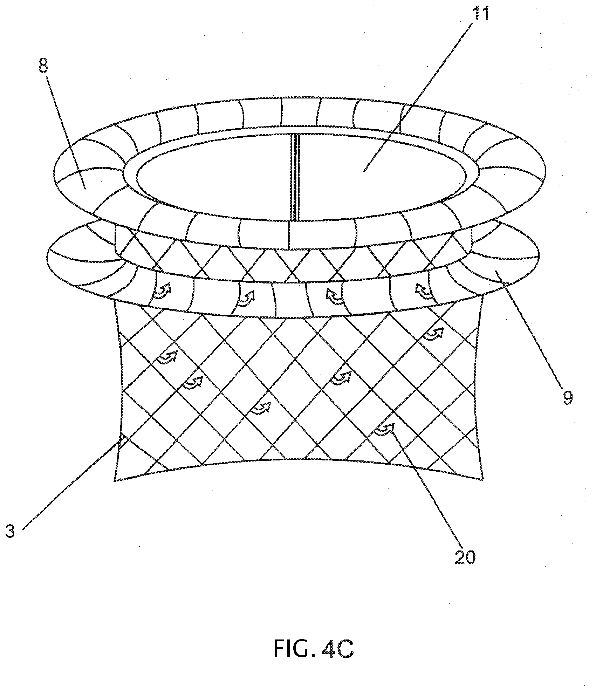

Figures 4A, 4B and 4C show the stents (3) in an open position, respectively, without valves, with the biological valve (10) and with the mechanical valve (11).

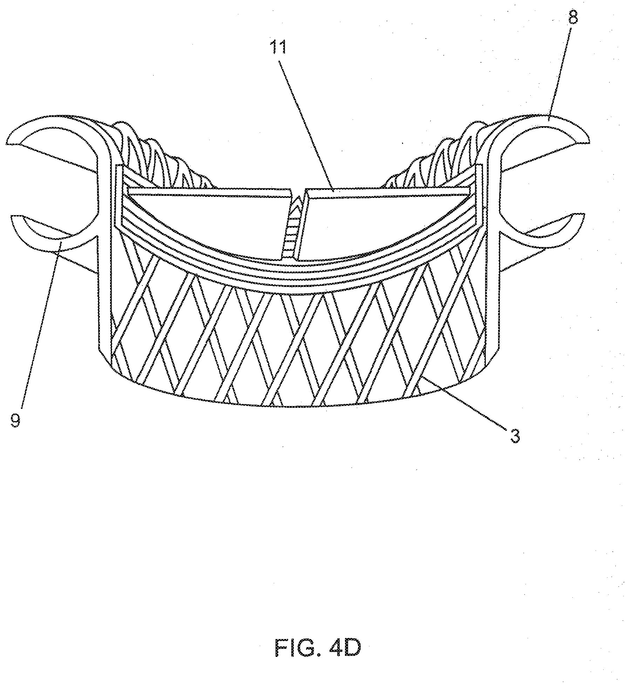

Figure 4D shows the device, in a cross-sectional view, with the mechanical valve (11) installed in the stent body (3) with the upper flap (8) and the lower flap (9) in the position of application to the heart valve.

### DESCRIPTION OF THE INVENTION

The device of this invention comprises a stent assembly for heart valve and application device thereof. The stent comprises a tubular structure with one of its ends including an upper flap (8) and a lower flap (9). When the stent (3) is released from the application device (1 or 2), the tubular structure expands, and the flaps are opened, fixing the stent (3) to the heart valve.

Due to its shape, the stent requires a specific application device (1 or 2) to be used. The application device (1 or 2) features a plunger (5) with two screws (6) that pierce through it. The adjustments of the screws (6), in one of the ends, are located on the outer part of the application device (1 or 2) and the threaded end (7) is on the inner part of the application device. In the threaded part (7), the stent (3) is attached and kept inside the application device, as seen in Figure 2C. Preferably, the application device must have an anatomical shape, which facilitates intra-arterial movement until the inside of the cardiac muscle.

There is the option of keeping the application device assembled and sealed by a cap (4), allowing the stent (3) to be kept in a preservation solution, such as formaldehyde, for example.

Whichever the procedure, a plasty ring may be featured or a previously inserted heart valve, which facilitates, in these situations, mechanical support of the stent. Depending on how the procedure is carried out, the stent must be placed within the application device (1 or 2). This is because the flaps (8 and 9) of the stent (3) have to be positioned in the heart valve, while the stent body must remain in the ventricular part of the valve.

In case of an applying procedure from the upper part of the myocardium, the stent (3) must be assembled with the flaps near the plunger (5). In the procedure, from the lower part of the myocardium, the stent (3) must be assembled with the flaps (8 and 9) closer to the exit of the application device (2). With these two assemblies, the correct position of the stent (3) is ensured inside the heart valve. Furthermore, the stent (3) must have a valve in the inner part of its tubular structure. This valve must enable the blood flow to occur only on one direction: always from the atrium to the ventricle, avoiding backflow in the region.

The valve is assembled in an adapter (12) which, in turn, is fixed to the stent body (3), preferably, between the flaps (8 and 9).

The valve may be made of biological material (10) or mechanical (11). In case of a biological valve, the stent (3) may be compressed within the application device (1 or 2) and kept inside with a preservative solution. However, when mechanical valves (11) are used, the stent (3) must be stored without the mechanical valve (11), with only the upper flap (8) and lower flap (9) being compressed within the application device (1 or 2).

Alternatively, the stent may be stored without the mechanical valve (11), with the mechanical valve-adapter (9) assembly fixed inside the stent (3) during the surgical procedure.

## Claims

1. STENT WITH MECHANICAL OR BIOLOGICAL HEART VALVE FOR MINIMALLY INVASIVE VALVE REPLACEMENT PROCEDURE, comprising a stent body (3) of tubular shape and grated structure,
wherein one of the ends of the stent body (3) has an upper flap (8) and a second lower flap (9), the second lower flap (9) being near and parallel to the upper flap (8) and positioned between the upper flap (8) and an intermediate part of the stent body (3), with both flaps (8 and 9) and the stent body (3) made of wired material and coated with inert material, which allows in use physiological movement of the mitral annulus and fitting an adapter (12) within the stent body (3), which is used to support a mechanical (11) or biological (10) heart valve within the stent body (3) between both flaps (8 and 9), and wherein the stent is positioned in an annuloplasty ring, previously positioned in the natural heart valve where the stent body (3) is to be placed, with the ring placed between the upper flap (8) and lower flap (9) on the outer part of the stent body (3), wherein said mechanical (11) or biological (10) valve is screwed to said adapter (12).

2. STENT, according to claim 1 wherein
the mechanical (11) or biological (10) heart valve is a mechanical heart valve (11) with two floodgates that allow a unidirectional flow.

3. STENT, according to claim 1
wherein the mechanical (11) or biological (10) heart valve is a biological (10) heart valve produced from a bovine or pig pericardium.

4. STENT, according to any of the previous
claims, wherein the material of the stent body (3) has a radiographic opaque property, allowing it to be viewed through radiography.

5. STENT, according to any of the previous
claims when dependent on claim 1, wherein small hooks (20) are placed on the upper flap (8), lower flap (9) and the stent body (3) that grapple the patient's muscle, facilitating the positioning and fixing of the stent body (3) to the annuloplasty ring.

6. STENT, according to the previous claims,
wherein the upper flap (8) and lower flap (9) are made of metallic segments in its structure that aid the mechanical resistance of the flaps.

7. SYSTEM comprising a stent according to
the previous claims and an application device (1, 2), wherein the application device (1, 2)comprises a cylindrical body (15), in which there is a plunger (5) which is pierced through by two screws (6) that fix and attach the stent body (3), said stent being stretched and elongated inside said cylindrical body (15), wherein said stent is positioned in such a way that the end of said stent body (3), the end where the upper flap (8) and lower flap (9) are placed, is near said plunger (5), so that in use the first part to come out from said application device (1) during an applying procedure from the upper part of the myocardium is the part of the stent body (3) opposite said end.

8. SYSTEM, according to claim 7, wherein said stent body (3) is positioned within the cylindrical body (15) with the end of the stent body (3) where the flaps (8 and 9) are placed being close to the exit of the application device (2) and released first in use, in an opposite way with respect to the position of claim 7, during an applying procedure from the lower part of the myocardium.

9. SYSTEM, according to claims 7 or 8,
wherein said application device (1, 2) has a thread, at the end where the stent comes out, that allows the placement of a cap (4) that seals the inner part of the cylindrical body (15) .

10. SYSTEM, according to any of claims 7-9
wherein said application device (1, 2) has a diameter large enough to keep said stent with mechanical valve (11) with only the flaps (8, 9) compressed within the application device.

## Patentansprüche

1. STENT MIT MECHANISCHER ODER BIOLOGISCHER HERZKLAPPE FüR MINIMAL-INVASIVES KLAPPEN-ERSATZVERFAHREN, umfassend einen Stentkörper (3) mit röhrenförmiger Form und Gitterstruktur,
worin eines der Enden des Stentkörpers (3) eine obere Klappe (8) und eine zweite untere Klappe (9) aufweist, wobei die zweite untere Klappe (9) nahe und parallel zur oberen Klappe (8) und zwischen der oberen Klappe (8) und einem Zwischenteil des Stentkörpers (3) positioniert ist, wobei beide Klappen (8 und 9) und der Stentkörper (3) aus Drahtmaterial bestehen und mit inertem Material beschichtet sind, das bei Verwendung die physiologische Bewegung des Mitralanulus und das Einpassen eines Adapters (12) in den Stentkörper (3) ermöglicht, der verwendet wird, um eine mechanische (11) oder biologische (10) Herzklappe innerhalb des Stentkörpers (3) zwischen den beiden Klappen (8 und 9) zu stützen, und worin der Stent in einem Anuloplastie-Ring positioniert ist, der zuvor in der natürlichen Herzklappe, in der der Stentkörper (3) platziert werden soll, positioniert wurde, wobei der Ring zwischen der oberen Klappe (8) und der unteren Klappe (9) auf dem äußeren Teil des Stentkörpers (3) angeordnet ist, worin die besagte mechanische (11) oder biologische (10) Klappe mit dem besagten Adapter (12) verschraubt ist.

2. STENT nach Anspruch 1, worin
die mechanische (11) oder biologische (10) Herzklappe eine mechanische Herzklappe (11) mit zwei Schleusen ist, die einen unidirektionalen Fluss ermöglichen.

3. STENT nach Anspruch 1,
worin die mechanische (11) oder biologische (10) Herzklappe eine biologische (10) Herzklappe ist, die aus einem Rinder- oder Schweineperikard hergestellt wird.

4. STENT nach irgendeinem der vorangegangenen
Ansprüche, worin das Material des Stentkörpers (3) eine röntgenopake Eigenschaft aufweist, wodurch er durch Radiographie betrachtet werden kann.

5. STENT nach irgendeinem der vorangegangenen
Ansprüche, wenn abhängig von Anspruch 1, worin kleine Haken (20) an der oberen Klappe (8), der unteren Klappe (9) und dem Stentkörper (3) angeordnet sind, die den Muskel des Patienten ergreifen, um die Positionierung und Fixierung des Stentkörpers (3) an dem Anuloplastie-Ring zu erleichtern.

6. STENT nach den vorangegangenen Ansprüchen,
worin die obere Klappe (8) und die untere Klappe (9) in ihrer Struktur aus Metallsegmenten bestehen, die die mechanische Festigkeit der Klappen unterstützen.

7. SYSTEM, umfassend einen Stent nach
den vorangegangenen Ansprüchen und eine Applikationsvorrichtung (1, 2), worin die Applikationsvorrichtung (1, 2) einen zylindrischen Körper (15) umfasst, in dem sich ein Kolben (5) befindet, der von zwei Schrauben (6) durchbohrt ist, die den Stentkörper (3) fixieren und befestigen, wobei der besagte Stent im Inneren des besagten zylindrischen Körpers (15) gestreckt und langgezogen ist, worin der besagte Stent solchermaßen positioniert ist, dass das Ende des besagten Stentkörpers (3), das Ende, an dem die obere Klappe (8) und die untere Klappe (9) angeordnet sind, sich nahe des besagten Kolbens (5) befindet, sodass bei Verwendung der erste Teil, der aus der besagten Applikationsvorrichtung (1) während eines Applikationsverfahrens aus dem oberen Teil des Myokards austritt, der Teil des Stentkörpers (3) gegenüber dem besagten Ende ist.

8. SYSTEM nach Anspruch 7, worin der besagte
Stentkörper (3) innerhalb des zylindrischen Körpers (15) positioniert ist, wobei das Ende des Stentkörpers (3), an dem die Klappen (8 und 9) angeordnet sind, nahe dem Ausgang der Applikationsvorrichtung (2) liegt und bei Verwendung, auf eine entgegengesetzte Weise in Bezug auf die Position von Anspruch 7, während eines Applikationsverfahrens vom unteren Teil des Myokards zuerst freigegeben wird.

9. SYSTEM nach Anspruch 7 oder 8,
worin die besagte Applikationsvorrichtung (1, 2) an dem Ende, an dem der Stent austritt, ein Gewinde aufweist, das die Anordnung einer Kappe (4), die den inneren Teil des zylindrischen Körpers (15) abdichtet, ermöglicht.

10. SYSTEM nach irgendeinem der Ansprüche 7-9,
worin die besagte Applikationsvorrichtung (1, 2) einen Durchmesser aufweist, der groß genug ist, um den besagten Stent mit mechanischer Klappe (11) nur mit den Klappen (8, 9), die innerhalb der Applikationsvorrichtung komprimiert sind, zu halten.

## Revendications

1. ENDOPROTHÈSE AVEC VALVE CARDIAQUE MÉCANIQUE OU BIOLOGIQUE POUR UNE PROCÉDURE MINI-INVASIVE DE REMPLACEMENT VALVULAIRE, comprenant un corps d'endoprothèse (3) de forme tubulaire et une structure à grille,
dans laquelle l'une des extrémités du corps de l'endoprothèse (3) comporte un rabat supérieur (8) et un deuxième rabat inférieur (9), le deuxième rabat inférieur (9) étant proche et parallèle au rabat supérieur (8) et positionné entre le rabat supérieur (8) et une partie intermédiaire du corps de l'endoprothèse (3), les deux rabats (8 et 9) et le corps de l'endoprothèse (3) étant constitués d'un matériau filaire et recouverts d'un matériau inerte, qui permet pendant l'utilisation le mouvement physiologique de l'anneau mitral et la mise en place d'un adaptateur (12) à l'intérieur du corps de l'endoprothèse (3), qui est utilisé pour soutenir une valve cardiaque mécanique (11) ou biologique (10) à l'intérieur du corps de l'endoprothèse (3) entre les deux rabats (8 et 9), et dans laquelle l'endoprothèse est positionnée dans un anneau d'annuloplastie, préalablement positionné dans la valve cardiaque naturelle où le corps de l'endoprothèse (3) doit être placé, avec l'anneau placé entre le rabat supérieur (8) et le rabat inférieur (9) sur la partie extérieure du corps de l'endoprothèse (3), dans laquelle ladite valve mécanique (11) ou biologique (10) est vissée audit adaptateur (12).

2. ENDOPROTHÈSE, selon la revendication 1, dans laquelle la valve cardiaque mécanique (11) ou biologique (10) est une valve cardiaque mécanique (11) avec deux vannes qui permettent un écoulement unidirectionnel.

3. ENDOPROTHÈSE, selon la revendication 1, dans laquelle la valve cardiaque mécanique (11) ou biologique (10) est une valve cardiaque biologique (10) produite à partir d'un péricarde bovin ou porcin.

4. ENDOPROTHÈSE, selon l'une des revendications précédentes, dans laquelle le matériau du corps de l'endoprothèse (3) a une propriété radiographique opaque, ce qui lui permet d'être visualisé par radiographie.

5. ENDOPROTHÈSE, selon l'une des revendications précédentes, lorsqu'elle dépend de la revendication 1, dans laquelle de petits crochets (20) sont placés sur le rabat supérieur (8), le rabat inférieur (9) et le corps de l'endoprothèse (3) qui saisissent le muscle du patient, facilitant le positionnement et la fixation du corps de l'endoprothèse (3) à l'anneau d'annuloplastie.

6. ENDOPROTHÈSE, selon les revendications précédentes, dans laquelle le rabat supérieur (8) et le rabat inférieur (9) sont constitués de segments métalliques dans leur structure qui contribuent à la résistance mécanique des rabats.

7. SYSTÈME comprenant une endoprothèse selon les revendications précédentes et un dispositif d'application (1, 2), dans lequel le dispositif d'application (1, 2) comprend un corps cylindrique (15), dans lequel se trouve un piston (5) qui est percé par deux vis (6) qui fixent et attachent le corps de l'endoprothèse (3), ladite endoprothèse étant étirée et allongée à l'intérieur dudit corps cylindrique (15), dans lequel ladite endoprothèse est positionnée de telle sorte que l'extrémité dudit corps d'endoprothèse (3), l'extrémité où le rabat supérieur (8) et le rabat inférieur (9) sont placés, est près dudit piston (5), de sorte que lors de l'utilisation, la première partie à sortir dudit dispositif d'application (1) au cours d'une procédure d'application de la partie supérieure du myocarde soit la partie du corps de l'endoprothèse (3) opposée à ladite extrémité.

8. SYSTÈME, selon la revendication 7, dans lequel ledit corps de l'endoprothèse (3) est positionné à l'intérieur du corps cylindrique (15) avec l'extrémité du corps de l'endoprothèse (3) où les rabats (8 et 9) sont placés étant proche de la sortie du dispositif d'application (2) et libérée en premier lors de l'utilisation, d'une manière opposée à la position selon la revendication 7, au cours d'une procédure d'application à partir de la partie inférieure du myocarde.

9. SYSTÈME, selon les revendications 7 ou 8, dans lequel ledit dispositif d'application (1, 2) a un filetage, à l'extrémité où sort l'endoprothèse, qui permet la mise en place d'un capuchon (4) qui ferme la partie intérieure du corps cylindrique (15).

10. SYSTÈME, selon l'une des revendications 7 à 9, dans lequel ledit dispositif d'application (1, 2) a un diamètre suffisamment grand pour maintenir ladite endoprothèse avec valve mécanique (11) avec seulement les rabats (8, 9) comprimés à l'intérieur du dispositif d'application.
